# EUROPÄISCHE PATENTSCHRIFT

(11) **EP 4 288 125 B1**
(45) Veröffentlichungstag und Bekanntmachung des Hinweises auf die Patenterteilung: **23.04.2025**
(21) Anmeldenummer: 22709206.1
(22) Anmeldetag: 02.02.2022
(51) Int. Cl.: A61L 31/10, A61L 31/08, A61L 31/14, A61L 33/00

(54) **BESCHICHTETES MEDIZINPRODUKT UND VERFAHREN ZUR BESCHICHTUNG EINES MEDIZINPRODUKTS**
COATED MEDICINAL PRODUCT AND METHOD FOR COATING A MEDICINAL PRODUCT
PRODUIT MÉDICINAL ENROBÉ ET PROCÉDÉ D'ENROBAGE D'UN PRODUIT MÉDICINAL

(30) Priorität: 02.02.2021 DE 102021102377
(43) Veröffentlichungstag der Anmeldung: 13.12.2023
(73) Patentinhaber: Phenox GmbH, 44801 Bochum (DE)
(72) Erfinder: LENZ-HABIJAN, Tim, 44627 Herne (DE); BANNEWITZ, Catrin, 44789 Bochum (DE); TRÖSKEN, Volker, 58456 Witten (DE); HANNES, Ralf, 44137 Dortmund (DE); MONSTADT, Hermann, 44797 Bochum (DE); HENKES, Hans, 70192 Stuttgart (DE)
(74) Vertreter: Schneiders & Behrendt Bochum
(86) Internationale Anmeldenummer: PCT/EP2022/052403
(87) Internationale Veröffentlichungsnummer: WO 2022/167447

(56) Entgegenhaltungen:
- WO-A1-2008/143933
- WO-A1-2018/210989

## Beschreibung

Die Erfindung betrifft eine Beschichtung für Medizinprodukte, wobei die Beschichtung die Zelladhärenz fördernde Eigenschaften aufweist.

Der Einsatz von Medizinprodukten zur Behandlung verschiedenster Krankheitsbilder nimmt stetig zu. Viele dieser Medizinprodukte stellen Implantate dar, die während der Behandlung dauerhaft in das Gefäßsystem oder allgemein in den Körper des Patienten gelangen, um dort zu verbleiben.

Damit ein solches Implantat nicht dauerhaft als Fremdkörper wahrgenommen wird, ist es von Vorteil, wenn das Implantat möglichst bald nach der Implantation einwächst. Hierzu müssen sich körpereigene Zellen an das Implantat anlagern. Von Vorteil ist daher ein Implantat, welches eine Beschichtung aufweist, die die Zelladhärenz fördert. Gleichzeitig sollte das Implantat kein inflammatorisches Potential aufweisen. Im Falle von Stents und ähnlichen Medizinprodukten, die in ein Blutgefäß implantiert werden, müssen Restenosen verhindert werden.

Eine Aminosäuresequenz, die die Zelladhäsion und die mechanische Verankerung von Zellen fördert, ist die RGD-Sequenz gemäß Einbuchstabencode, die sich aus den Aminosäuren Arginin, Glycin und Asparaginsäure zusammensetzt. Diese Sequenz kommt in Proteinen der extrazellulären Matrix, beispielsweise Fibronektin und Vitronektin vor. Mit Hilfe von Integrinen (Zelloberflächen-Rezeptoren) sind Zellen in der Lage, an diese Aminosäuresequenz zu binden. Es sind auch andere Peptidsequenzen mit Integrin-bindenden Motiven bekannt.

Ein Stent mit verbesserten Re-Endothelisierungseigenschaften wird in der WO 2008/143933 A1 beschrieben. Obgleich die Bindung von Integrin-selektiven Peptiden an Integrine auf der Zelloberfläche eine Signalkaskade aktiviert, die die Proliferation der Zelle induzieren und damit eine Restenose bewirken könnte, haben die Erfinder herausgefunden, dass trotz der Integrinaktivierung das RGD-Peptid einer Restenose gerade entgegenwirkt. Dies wird auf eine verstärkte Adhäsion von Endothelzellen am Stent zurückgeführt, die für eine rasche Endothelisierung und ein Einwachsen des Stents in das umgebende Gewebe sorgt, wodurch wiederum die Ausbildung von Restenosen vermieden wird.

Bei Implantaten, die in Blutgefäße eingebracht werden, ist jedoch die Vermeidung von durch das Implantat selbst ausgelöster Blutgerinnung, d. h. die Vermeidung von Thrombozytenadhäsion und Thrombozytenaggregation von entscheidender Bedeutung. Eine solche und somit die Bildung von Blutgerinnseln bzw. Thromben kann bei dauerhaften oder temporären Implantaten ebenso beobachtet werden wie bei medizinischen Instrumenten, die nur kurzzeitig zur Behandlung oder zu Diagnosezwecken in den Körper des Patienten eingeführt werden. Die Thrombozyten haften auf der durch körpereigene Proteine markierten Oberfläche des eingeführten Medizinprodukts (Thrombozytenadhäsion), wodurch es in der Folge zur Bildung eines Thrombus kommen kann (Thrombozytenaggregation).

Löst sich solch ein Thrombus beispielsweise im Gefäßsystem, kann es zu teils schwerwiegenden bis hin zu tödlichen Folgeerkrankungen kommen. Dies ist vor allem dann der Fall, wenn der losgelöste Thrombus fortgespült wird und sich als Embolus in kleineren Gefäßen festsetzt, diese weitestgehend verschließt und so die ausreichende Versorgung der sich anschließenden Bereiche gefährdet. Resultierende Krankheitsbilder können beispielsweise Schlaganfälle, Herzinfarkte oder Thrombosen sein.

Um diese Risiken für den Patienten zu minimieren, wird heutzutage in der Regel im Rahmen einer Operation beziehungsweise Intervention auf die duale Plättchenhemmung zur Reduzierung des Thromboserisikos zurückgegriffen. Dabei erhält der Patient in der Regel vor dem Eingriff eine erste Dosis einer Kombination von Thrombozytenaggregationshemmern, insbesondere einer Kombination von Acetylsalicylsäure (ASS) und Clopidogrel, und muss diese auch nach dem Eingriff noch für eine bestimmte Zeit regelmäßig einnehmen. Daneben können auch andere Thrombozytenaggregationshemmer zum Einsatz kommen, die in den entsprechenden Leitlinien zur dualen Plättchenhemmung genannt sind. In der Regel werden diese anstelle des Clopidogrels in Kombination mit ASS verwendet. Beispiele sind Prasugel oder Ticagrelor.

Der Nachteil der dualen Plättchenhemmung liegt darin, dass sie systemisch erfolgt und somit auch systemisch wirkt. Für die Dauer der dualen Plättchenhemmung ist somit das Blutungsrisiko für den Patienten insgesamt erhöht.

Ein weiterer Nachteil besteht darin, dass die duale Plättchenhemmung für einige Patientengruppen gar nicht möglich ist, da für diese die mit der dualen Plättchenhemmung verbundenen Risiken, insbesondere das erhöhte Blutungsrisiko, so groß sind, dass diese die Vorteile von vornherein überwiegen. Im Extremfall stehen manchen Patienten bestimmte Behandlungsmethoden allein aufgrund der Tatsache nicht zur Verfügung, dass sie nach einem Eingriff nicht ausreichend gegen ein Thromboserisiko geschützt werden können. Die duale Plättchenhemmung ist beispielsweise Standard nach der Implantation endovaskulärer Prothesen (Stents und dergleichen). Aus diesem Grund wurde in der WO 2018/210989 A1 vorgeschlagen, Medizinprodukte mit erst bei der Bindung an das Substrat oligo- oder polymerisierenden Sacchariden zu beschichten. Diese imitieren die natürliche Glykokalyx, die die Zellen der Blutgefäße mit einer Art Schleimschicht überzieht und aus verschiedenen Polysacchariden besteht, die kovalent an die Membranproteine (Glycoproteine) und Membranlipide (Glycolipide) gebunden sind. Es handelt sich somit um einen biomimetischen Effekt.

Es stellt sich somit die Aufgabe, Medizinprodukte zur Verfügung zu stellen, die ein rasches Einwachsen des Medizinprodukts in das umgebende Gewebe durch Verstärkung der Zelladhäsion bzw. Zelladhärenz gewährleisten, gleichzeitig aber eine verstärkte Thrombozytenadhäsion und -aggregation verhindern.

Die Aufgabe wird erfindungsgemäß gelöst durch ein endovaskuläres Medizinprodukt umfassend eine mindestens teilweise Beschichtung, wobei das Medizinprodukt ein für den dauerhaften Verbleib im Körper vorgesehenes Implantat ist und wobei die Beschichtung eine Funktionsschicht umfasst und die Funktionsschicht Saccharide und Peptidsequenzen mit Integrin-bindenden Motiven enthält.

Die Erfindung beruht auf dem Gedanken, auf der einen Seite die Zelladhäsion und das Einwachsen zu fördern, auf der anderen Seite aber die Adhäsion eines bestimmten Zelltyps, nämlich die Thrombozytenadhäsion zu vermeiden. Es hat sich herausgestellt, dass dies möglich ist, wenn man nicht lediglich Peptidsequenzen mit Integrin-bindenden Motiven auf das Medizinprodukt aufbringt, sondern zudem Saccharide in die Funktionsschicht integriert.

Das erfindungsgemäße Medizinprodukt umfasst im Wesentlichen zumindest ein Substrat als Basis des eigentlichen Medizinprodukts sowie eine Funktionsschicht. Die Funktionsschicht verleiht dem Medizinprodukt die gewünschten Eigenschaften.

Als Peptidsequenz mit Integrin-bindendem Motiv besonders bevorzugt ist die RGD-Peptidsequenz, deren Vermittlung der Zelladhäsion gut untersucht ist. Denkbar ist jedoch auch die Verwendung weiterer Peptidsequenzen mit Integrin-bindendem Motiv. Soweit im Rahmen der Anmeldung von Peptiden oder Peptidsequenzen die Rede ist, sind darunter solche Peptide/Peptidsequenzen zu verstehen, die über mindestens ein Integrin-bindendes Motiv verfügen, soweit sich aus dem Zusammenhang nichts anderes ergibt.

Um eine Bindung der Saccharide und der Peptidsequenzen an der Oberfläche des Medizinprodukts herbeizuführen, findet vorzugsweise eine Polymerisation der Saccharide und eine Reaktion mit den Peptiden zur Ausbildung der Funktionsschicht statt. Hierzu werden sowohl die Saccharide als auch die Peptide mit polymerisierbaren oder reaktiven Gruppen funktionalisiert, die in der Lage sind, an die Oberfläche des Medizinprodukts zu binden und eine Polymerisation herbeizuführen. Die Peptide müssen nicht unbedingt in die Polymerisation selbst einbezogen sein, es ist ausreichend, wenn diese über reaktive Gruppen verfügen, über die eine Verknüpfung mit den Sacchariden erfolgen kann. Häufig werden jedoch die polymerisierbaren Gruppen der Saccharide und die reaktiven Gruppen der Peptide identisch gewählt.

Hierzu können Saccharide einerseits und Peptide andererseits jeweils für sich an polymerisierbare/reaktive Gruppen gebunden werden, wobei sodann eine Reaktion, die in der Regel zumindest teilweise eine Polymerisation ist, zur Ausbildung der Funktionsschicht stattfindet. Die polymerisierbaren/reaktiven Gruppen, mit denen Saccharide bzw. Peptide versehen werden, können dabei identisch oder zumindest so beschaffen sein, dass eine Reaktion untereinander stattfinden kann. Entsprechend ist eine Copolymerisation möglich, bei der Reaktionen untereinander erfolgen. Ebenso möglich sind jedoch getrennte Reaktionen von funktionalisierten Sacchariden und Peptiden an der Oberfläche des Medizinprodukts. Zum Durchführen einer Polymerisation kann eine Lösung, die sowohl funktionalisierte Saccharide als auch funktionalisierte Peptide enthält, auf das Substrat aufgebracht werden, sodass die Ausbildung der Funktionsschicht aus einer Lösung heraus erfolgt. Funktionalisierte Saccharide und funktionalisierte Peptide können dabei in unterschiedlichem Verhältnis vorliegen.

Die Polymerisation oder Reaktion der polymerisierbaren/reaktiven Gruppen, mit denen Saccharide bzw. Peptide versehen sind, muss jedoch nicht zeitgleich erfolgen, es ist auch möglich, dass zunächst eine Reaktion der einen Stoffgruppe erfolgt, bevor sodann reaktive Einheiten der anderen Stoffgruppe aufgebracht werden. Es handelt sich somit um zwei (oder mehr) aufeinanderfolgende Umsetzungen der verschiedenen mit polymerisierbaren bzw. reaktiven Gruppen ausgestatteten Verbindungen, nämlich Saccharide einerseits und Peptide andererseits. Man spricht in diesem Zusammenhang auch von Postpolymerisation. Praktisch geht man in der Regel so vor, dass man zunächst eine Polymerisation aus einer ersten Lösung mit der ersten polymerisierbaren Stoffgruppe durchführt, anschließend die Lösungen wechselt und eine Umsetzung aus der zweiten Lösung mit der zweiten polymerisierbaren bzw. reaktiven Stoffgruppe vornimmt. Auch bei dem beschriebenen Prinzip der Postpolymerisation können funktionalisierte Saccharide und funktionalisierte Peptide in unterschiedlichem Verhältnis zum Einsatz kommen.

Beim Einsatz getrennter Moleküle p-Saccharid einerseits und p-Peptid andererseits, wobei p für eine beliebige polymerisierbare oder allgemeiner reaktive Gruppe steht, ergeben sich somit verschiedene Varianten, von denen hier einige beispielhaft angeführt sind:
a) Zeitgleiche Polymerisation von p-Saccharid und p-Peptid
b) Zunächst Polymerisation von p-Saccharid, anschließend Austausch der Lösungen und Reaktion mit p-Peptid
c) Zunächst Umsetzung mit bzw. Polymerisation von p-Peptid, anschließend Austausch der Lösungen und Umsetzung mit bzw. Polymerisation von p-Saccharid
d) Zunächst Polymerisation von p-Saccharid, anschließend Austausch der Lösungen und Reaktion mit p-Peptid, anschließend erneuter Austausch der Lösungen und Polymerisation von bzw. Reaktion mit p-Saccharid

In entsprechender Weise sind prinzipiell beliebige andere Kombinationen aufeinanderfolgender Polymerisationen bzw. Umsetzungen denkbar.

Da die räumliche Ausdehnung des Peptids deutlich über die eines Monosaccharids hinausgeht, muss in der Regel eine größere Menge des funktionalisierten Saccharids polymerisiert oder umgesetzt werden als funktionalisiertes Peptid. Dies gilt für eine zeitgleich durchgeführte Polymerisation ebenso wie für zeitlich aufeinanderfolgende Postpolymerisationen. Auf diese Weise wird erreicht, dass sich an der Oberfläche des beschichteten Medizinprodukts die positiven Eigenschaften des Saccharids und des Peptids gleichermaßen bemerkbar machen. Alternativ kann, wie im obigen Beispiel d), eine zusätzliche Menge p-Saccharid aufgebracht werden, um die Menge an auf der Oberfläche wirksamem Saccharid bewusst zu erhöhen.

Ein Beispiel für ein RGD-Peptid, das über eine funktionelle Gruppe zur weiteren Umsetzung mit Polymeren bzw. polymerisierbaren Verbindungen verfügt, ist
Acetyl-Cys-Doa-Doa-Gly-Arg-Gly-Asp-Ser-Pro-NH₂
mit Doa = 8-Amino-3,6-dioxaoctansäure
wie es von der Firma Cellendes GmbH, Reutlingen/Deutschland bezogen werden kann. Die Funktionalisierung erfolgt über die Thiolgruppe. Über diese kann beispielsweise eine Kopplung mit Polyvinylalkohol erfolgen, das wiederum mit Polyethylenglycol quervernetzt werden kann. Die Polyethylenglycoleinheit kann auch über eine polymerisierbare Acrylatfunktion verfügen.

Ein weiteres RGD-Peptid, das erfindungsgemäß verwendet werden kann, ist GRGDSPK (Gly-Arg-Gly-Asp-Ser-Pro-Lys), welches über eine reaktive Acrylatfunktion verfügen kann. Die Beschichtung mit diesem Peptid, das die Sequenz RGD enthält, kann insbesondere in der Form erfolgen, dass zunächst eine Polymerisation von p-Saccharid auf der zu beschichtenden Oberfläche vorgenommen wird, anschließend ein Austausch der Lösungen gegen die RGD-Peptid-Lösung erfolgt und sodann die Umsetzung mit dem funktionalisierten GRGDSPK stattfindet.

Eine weitere Möglichkeit besteht darin, polymerisierbare Moleküle vorzusehen, die sowohl das Saccharid als auch das Peptid tragen. Dies kann auch in der Weise geschehen, dass die polymerisierbare Gruppe mit dem Saccharid und das Saccharid mit dem Peptid verknüpft ist bzw. umgekehrt. Bei Verwendung eines Acrylatlinkers wäre ein Beispiel:
Acrylat-Saccharid-RGD-Peptid

Bei der Polymerisation zur Ausbildung der Funktionsschicht entsteht damit automatisch eine Schicht, die beide Funktionalitäten aufweist. Allerdings kann auch bei Verwendung polymerisierbarer Moleküle mit Saccharid- und Peptideinheit zusätzlich p-Saccharid oder p-Peptid gleichzeitig, nachträglich oder zuvor aufgebracht werden, um die Eigenschaften der Funktionsschicht gezielt zu beeinflussen. Aufgrund der oben bereits erwähnten sterischen Verhältnisse zwischen Peptid einerseits und Saccharid andererseits ist es in der Regel sinnvoll, zusätzliches p-Saccharid einzusetzen.

Bei der Verwendung polymerisierbarer Moleküle, die sowohl das Saccharid als auch das Peptid tragen (p-Saccharid-Peptid, wobei p für eine beliebige polymerisierbare/reaktive Gruppe steht, unabhängig davon, mit welcher Funktionalität die polymerisierbare/reaktive Gruppe verknüpft ist) ergeben sich somit verschiedene Varianten, von denen hier einige beispielhaft angeführt sind:
a) Zeitgleiche (Co)polymerisation von p-Saccharid-Peptid und p-Saccharid
b) Zunächst Polymerisation von p-Saccharid, anschließend Austausch der Lösungen und Polymerisation von p-Saccharid-Peptid
c) Zunächst Polymerisation von p-Saccharid-Peptid, anschließend Austausch der Lösungen und Polymerisation von p-Saccharid

Zwischen der polymerisierbaren Gruppe und dem Saccharid bzw. Peptid kann ein Spacer vorhanden sein. Beispielsweise ist die Verwendung einer Polyethylenglycol (PEG)-Einheit möglich. Das Molekulargewicht der PEG-Einheit liegt vorzugsweise bei 300 bis 15.000 Da, insbesondere 1.000 bis 10.000 Da bzw. 2.000 bis 5.000 Da.

Soweit im Rahmen dieser Erfindung von RGD-Peptiden die Rede ist, sind hiermit Peptide gemeint, die eine RGD-Peptidsequenz aufweisen, bestehend aus Arginin, Glycin und Asparaginsäure (Arg-Gly-Asp). Die Peptidsequenz kann auch in cyclisierter Form vorliegen (cRGD). Das RGD-Peptid kann als Tripeptid vorliegen, es kann jedoch auch insgesamt aus einer größeren Zahl an Aminosäuren bestehen; wichtig ist jedoch, dass die Sequenz aus Arginin, Glycin und Asparaginsäure enthalten ist, welche für die Bindung der Integrine der Zellen, die sich an das Medizinprodukt anlagern sollen, von Bedeutung ist. Entsprechend kann das RGD-Peptid z. B. die Sequenz
Aₚ-RGD-B_{q}
aufweisen, wobei A und B unabhängig voneinander natürliche oder unnatürliche Aminosäuren sind und p und q unabhängig voneinander 0, 1, 2, 3, 4, 5, 6, 7, 8, 9 oder 10 sein können. Sowohl A als auch B können innerhalb der Peptidkette variieren.

Ebenfalls möglich ist ein zyklisches Peptid mit einer RGD-Sequenz wie z. B.
(-RGD-Zᵣ-),
wobei wiederum Z identische oder unterschiedliche, natürliche oder unnatürliche Aminosäuren sind und r = 0, 1, 2, 3, 4, 5, 6, 7, 8, 9 oder 10 ist.

Bei den Aminosäuren handelt es sich in der Regel um die natürlichen L-Aminosäuren, wobei eine Verwendung von einzelnen oder ausschließlich D-Aminosäuren erfindungsgemäß nicht ausgeschlossen ist. Auch Peptide, die eine RGD-Gruppe tragen, wobei Aminosäuren oder die Peptidbackbone funktionalisiert sind bzw. weitere Gruppen tragen, werden als RGD-Peptide im Sinne der Erfindung angesehen.

Die Oberflächenbelegung mit den Integrin-bindende Motive enthaltenden Peptidsequenzen erfolgt vorteilhafterweise so, dass zwar die Zelladhärenz für die Endothelzellen gesteigert wird, nicht aber die der deutlich kleineren Thrombozyten. Während Endothelzellen eine Größe von ca. 10 bis 25 µm aufweisen, haben Thrombozyten eine Größe von ca. 1 bis 4 µm. Bei nicht zu hoher Dichte der als Ankerpunkte dienenden Peptide haben daher zwar die Endothelzellen bereits ausreichend viele Peptidkontakte, um eine Zellreaktion auszulösen, die eine Adhärenz hervorruft, nicht jedoch die Thrombozyten. Wenn diese beispielsweise lediglich mit einzelnen Peptiden in Kontakt treten, ist das vermittelte Signal noch nicht ausreichend, eine Adhärenz der Thrombozyten hervorzurufen. Ohne sich an eine bestimmte Theorie binden zu wollen, ist dies vermutlich darauf zurückzuführen, dass bei geringer Dichte der Ankerpunkte eine initiale Adhärenz über den Integrinrezeptor zwar theoretisch noch möglich ist, die Abstände jedoch zu groß sind für eine über Integrinclusterbildung vermittelte Zellaktivierung.

Aus Untersuchungen auf mit RGD-Peptiden versehenen Oberflächen hat sich ergeben, dass der Abstand der immobilisierten Peptidliganden vorteilhafterweise mindestens 40 nm, weiter vorteilhaft mindestens 50 nm, insbesondere mindestens 60 nm beträgt. Als besonders vorteilhaft hat sich ein Abstand der Peptidliganden von bis zu 500 nm herausgestellt. Ein solcher Abstand der Peptidliganden sorgt auf der einen Seite für eine ausreichende Anlagerung und Adhärenz von Endothelzellen, während dies für die Thrombozyten noch nicht zu beobachten ist. Entsprechend wird das Ziel erreicht, die Anlagerung von Endothelzellen und das Einwachsen eines Medizinprodukts, insbesondere eines Implantats zu fördern, ohne jedoch das Risiko der Thrombozytenaggregation zu erhöhen. Durch entsprechende Wahl der für die Beschichtungsreaktionen verwendeten Reaktionsbedingungen wie Konzentration, Zeit, Temperatur, Auswahl des funktionalisierten Peptids etc. lässt sich die gewünschte Oberflächenbelegung gezielt einstellen. Diese kann bspw. mittels Rasterelektronen- oder Rasterkraftmikroskopie bestimmt werden.

Vorzugsweise handelt es sich bei den Sacchariden, die mit einer polymerisierbaren Gruppe verknüpft sind, um Monosaccharide. Unter Sacchariden werden in diesem Zusammenhang auch Reduktions- und Oxidationsprodukte von Sacchariden aufgefasst, insbesondere Zuckeralkohole. Erst bei der Bindung an das Substrat erfolgt eine Oligo- oder Polymerisation. Die funktionalisierten Monosaccharide werden kovalent an das Substrat gebunden, wobei die Gruppen, mit denen die Monosaccharide verknüpft sind, so ausgestaltet sind, dass eine kovalente Bindung an das Substrat erfolgen kann. Die Funktionsschicht umfasst somit eine komplexe Matrix, die durch die Polymerisation der aufgebrachten, funktionalisierten Monosaccharide sowie der Umsetzung mit den funktionalisierten Peptiden entstanden ist.

Bevorzugt handelt es sich bei den Sacchariden in ihrer nicht funktionalisierten Form mindestens teilweise um Zuckeralkohole oder entsprechende Derivate bzw. Isomere. Bei Zuckeralkoholen (Alditolen) handelt es sich um Reduktionsprodukte von Zuckern, bei denen eine Aldehydfunktion zum Alkohol reduziert wurde.

Ein bevorzugter Zuckeralkohol der Funktionsschicht entspricht in seiner nicht funktionalisierten Form einem Zuckeralkohol mit der Summenformel C₆H₁₄O₆, beispielsweise Sorbitol (Sorbit), und/oder dessen Derivate, beispielsweise Sorbitan. Andere Zuckeralkohole können sein Mannit (Mannitol), Lactit, Xylit (Xylitol), Threit, Erythrit oder Arabit. Die Struktur von Sorbitol wird nachfolgend wiedergegeben:

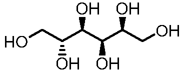

"In seiner nicht funktionalisierten Form" bedeutet, dass die angegebene Summenformel die Summenformel des nicht funktionalisierten Zuckeralkohols darstellt, jedoch gegebenenfalls auch dessen Derivate und/oder dessen Isomere umfassen soll.

Als Derivate sind im Sinne der Erfindung neben der allgemein in der Chemie üblichen Definition des Begriffs als "abgeleiteter Stoff ähnlicher Struktur" alle von dem Stoff durch Dehydratisierung ableitbaren cyclischen und heterocyclischen Verbindungen zu verstehen. Ein Beispiel hierfür ist das Sorbitan bzw. Sorbitananhydrid, das durch Abspaltung eines Wassermoleküls vom Sorbitol entsteht. Es stellt somit das Anhydrid von Sorbitol dar. Ein weiteres Beispiel ist das durch Abspaltung eines zusätzlichen Wassermoleküls erhältliche Isosorbid.

Die polymerisierbaren Gruppen, über die die Saccharide und Peptide funktionalisiert sind, können reaktive Mehrfachbindungen, insbesondere reaktive Doppelbindungen aufweisen. Die Polymerisation kann somit über die Doppelbindungen stattfinden. Insbesondere kann es sich um eine Acryl- oder Methacrylgruppe handeln, deren Eignung für Polymerisationsreaktionen dem Fachmann bekannt ist. Ebenso möglich ist der Einsatz anderer für eine Polymerisation geeigneter Gruppen wie Vinyl oder Allyl. Hinsichtlich des Peptids kann die polymerisierbare Gruppe, insbesondere ein (Meth)acrylatlinker, beispielsweise am N-Terminus angebracht sein. Die Oligo- oder Polymerisation der Saccharide erfolgt somit normalerweise über die polymerisierbaren Gruppen, über die die Saccharide funktionalisiert sind, hingegen findet in der Regel keine Neubildung von glycosidischen Bindungen statt.

Die Lösung, aus der die Funktionsschicht der erfindungsgemäßen Beschichtung aufgebaut wird, kann demnach einzelne oder mehrere der nachfolgenden Stoffe als Saccharidkomponente umfassen:
(1) Sorbitol-Acrylate (mit einer oder mehreren Acrylatgruppe/n), wobei sich die Acrylatgruppeln an verschiedenen Positionen befinden können. 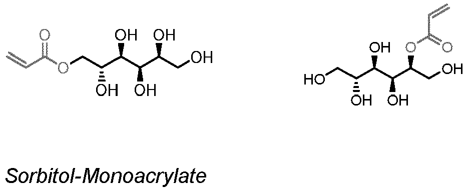 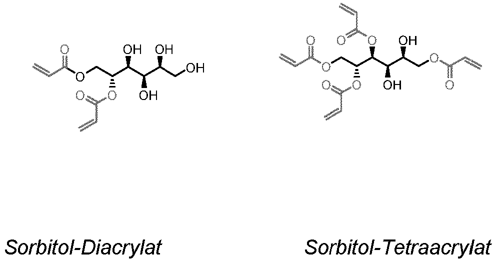
(2) Sorbitol-Acrylate (mit einer oder mehreren Acrylatgruppe/n), wobei die Sorbitol-Acrylate teilweise oxidiert sein können und eine Aldehyd-, Keto- und/oder eine Carboxygruppe umfassen können. 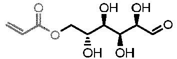 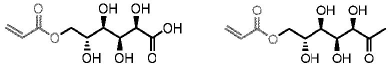
(3) Sorbitol-Acrylate (mit einer oder mehreren Acrylatgruppe/n), wobei diese weitere reaktive Gruppen umfassen können, beispielsweise Carboxygruppen. 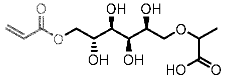
(4) Anhydride, beispielsweise Sorbitan-(Mono)-Acrylat mit einer polymerisierbaren Gruppe. 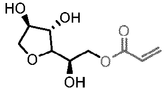
(5) Sorbitol mit einer nicht polymerisierbaren Gruppe, beispielsweise einer Carboxygruppe. 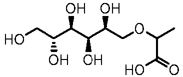
(6) Komplexe Sorbitol Verbindungen, die zwar nicht polymerisierbar sind, jedoch in die Polymermatrix der Funktionsschicht eingebaut werden können.

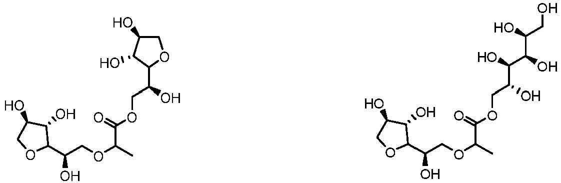

Die Struktur der Funktionsschicht kann über die konkrete Zusammensetzung der Stoffe variiert werden. So ist es beispielsweise möglich, engmaschigere Funktionsschichten durch einen erhöhten Anteil an Vernetzern zu erzeugen oder eher gering vernetzte Funktionsschichten mit längeren linearen Bereichen durch einen geringeren Anteil an Vernetzern.

Gemäß der Erfindung befindet sich auf dem Medizinprodukt selbst, welches das Substrat darstellt, zumeist noch eine Trägerschicht, die Haftvermittler umfasst, über die die Funktionsschicht mit dem Substrat verbindbar ist. Bevorzugte Haftvermittler im Sinne der Erfindung sind Silanhaftvermittler. Alternativ können auch andere Haftvermittler, beispielsweise polyolefinische Haftvermittler oder Haftvermittler auf Basis von Titanaten oder Zirkonaten zum Einsatz kommen.

Weitere Beispiele für Haftvermittler sind
- Thiole und Dithioverbindungen, besonders geeignet für Edelmetallsubstrate
- Amine und Alkohole, besonders geeignet für Platinsubstrate
- Carbonsäuren, besonders geeignet für Silbersubstrate und Aluminiumsubstrate, wobei das Aluminium eine Aluminiumoxidoberfläche aufweisen kann
- Phosphonsäuren (Phosphonate), besonders geeignet für Eisen-, Eisenoxid-, Titan- und Titandioxidsubstrate
- Komplexbildende Haftvermittler, insbesondere Chelate, die zum Teil auch nicht-kovalent an Substrate binden, besonders geeignet für verschiedene Metall- und Metalloxidsubstrate

Die Haftvermittler sollten über funktionelle Gruppen verfügen, über die eine Reaktion des Haftvermittlers mit der Funktionsschicht und damit eine in der Regel kovalente Ankopplung ermöglicht wird. Je nach Material des Medizinprodukts kann auch die Bindung des Haftvermittlers an das Medizinprodukt kovalent sein.

Eine geeignete Haftvermittlung kann beispielsweise über eine Silanisierung erfolgen, also eine chemische Anbindung von Silicium-, insbesondere Silanverbindungen an zumindest Teilen ihrer Oberfläche. An Oberflächen binden Silicium- und Silanverbindungen beispielsweise an Hydroxy- und Carboxy-Gruppen.

Unter einer Silanverbindung im Sinne der Erfindung sind all jene Verbindungen zu verstehen, die der allgemeinen Formel RₘSiXₙ folgen (m, n = 0-4, wobei R für organische Reste, insbesondere Alkyl-, Alkenyl- oder Arylgruppen, und X für hydrolysierbare Gruppen, insbesondere OR, OH oder Halogen mit R = Alkyl, Alkenyl oder Aryl steht. Insbesondere kann das Silan die allgemeine Formel RSiX₃ haben. Darüber hinaus gehören entsprechende Verbindungen mit mehreren Siliciumatomen zu den Silanverbindungen im Sinne der Erfindung. Insbesondere Silan-Derivate in Form von siliciumorganischen Verbindungen werden als Silanverbindungen im Sinne der Erfindung aufgefasst. Unter Silanverbindungen im Sinne der Erfindung sind entsprechend nicht nur solche Stoffe zu verstehen, die aus einem Silicium-Grundgerüst und Wasserstoff bestehen und die Bezeichnung Silane tragen.

Auch Polyolefine können als Haftvermittler eingesetzt werden, darunter chlorierte Polyolefine (CPO) oder acrylierte Polyolefine (APO).

Das Medizinprodukt, welches das beschichtbare Substrat bildet, kann aus verschiedenen Materialien gefertigt sein. Darunter fallen beispielsweise Metalle wie Nickel, Titan, Platin, Iridium, Gold, Kobalt, Chrom, Aluminium, Eisen oder Legierungen sowie Kombinationen hiervon. Beispielsweise kann auch ein Metall mit einem anderen Metall beschichtet werden, wobei auf die äußere Metallschicht wiederum die erfindungsgemäße Beschichtung aufgebracht wird, vorzugsweise bestehend aus der Trägerschicht und der Funktionsschicht. Unter beschichtbaren Metallen werden auch solche Substrate verstanden, bei denen das eigentliche Metall von einer Oxidschicht bedeckt ist. Weitere beschichtbare Substrate sind Gläser.

Eine besonders bevorzugte Ausführungsform betrifft ein Medizinprodukt, das ganz oder teilweise eine Goldbeschichtung trägt, durch welches die Röntgensichtbarkeit sichergestellt wird. Insbesondere kann auf diese Weise die Aufweitung des Medizinprodukts im Blutgefäß beobachtet werden, sodass der behandelnde Arzt erkennen kann, ob die Aufweitung in der gewünschten Form erfolgt. Dies ist z. B. bei einem Implantat zur Behandlung eines Vasopasmus von besonderem Vorteil. Auf die Goldbeschichtung wiederum wird dann die erfindungsgemäße Beschichtung aufgebracht, wobei die Funktionsschicht Saccharide und Peptidsequenzen mit Integrin-bindenden Motiven enthält. In den meisten Fällen kommt auch eine Trägerschicht zum Einsatz. Das Basismaterial des Medizinprodukts, auf das die Goldbeschichtung aufgebracht wird, kann ein übliches Metall bzw. eine übliche Metalllegierung für entsprechende Medizinprodukte sein, beispielsweise eine Nickel-Titan-Legierung, eine Kobalt-Chrom-Legierung oder Edelstahl.

Auch die Fertigung der Medizinprodukte aus verschiedenen Kunststoffen ist möglich, wie beispielsweise Polyamide (PA), Polytetrafluorethylen (PTFE), expanded Polytetrafluorethylen (ePTFE), Polylactide (PLA), Polyester, Polyether, Polyurethan, Polyolefine, weiterhin entsprechende BlockCopolymere. Dem Fachmann ist eine Vielzahl an geeigneten Kunststoffen im Bereich der Medizintechnik bekannt. Während bei metallischen oder oxidischen Oberflächen in der Regel ein Haftvermittler erforderlich ist, kommt ein solcher bei Polymeren als Substrat nicht immer zum Einsatz.

Die Erfindung ist jedoch nicht auf Beschichtungen der genannten Kunststoffe und Metalle beschränkt, vielmehr sind diese nur beispielhaft genannt. Prinzipiell richtet sich die Erfindung auf Beschichtungen aller denkbaren Materialien, die für entsprechende Medizinprodukte in Frage kommen.

Die Matrix der Funktionsschicht ist bevorzugt kovalent an die Trägerschicht oder das Substrat gebunden und wird bevorzugt mittels Pfropfpolymerisation synthetisiert, wobei die Funktionsschicht auf der Trägerschicht bzw. dem Substrat erzeugt wird. Die Polymerisation der aufgebrachten polymerisierbaren Gruppen, die die Saccharide und Peptide tragen, erfolgt vorzugsweise im Wesentlichen erst auf der Trägerschicht/dem Substrat beziehungsweise innerhalb der Funktionsschicht.

Als erfindungsgemäß werden unterschiedliche Arten der (Pfropf)polymerisation verstanden. Insbesondere kann ausgehend von einer Hauptkette das Wachstum der Seitenketten starten. Dieser Ansatz wird auch als "grafting from" bezeichnet. Ebenso ist es möglich, dass die Seitenketten bereits mit der Oligo- oder Polymerisation begonnen haben und die bereits wachsenden Seitenketten sich mit der Hauptkette verbinden ("grafting onto"). Schließlich können sich auch bereits oligo- oder polymerisierte Haupt- und Seitenketten zusammenfinden ("grafting through").

Die Funktionsschicht umfasst bevorzugt im Wesentlichen eine komplexe, stark verzweigte, hydrophile Matrix umfassend eine Vielzahl von Molekülen mit jeweils einer Hauptkette als polymerem Rückgrat und jeweils mehreren Seitenketten. Die Haupt- und/oder Seitenketten können mit weiteren Haupt- und/oder Seitenketten Bindungen eingehen. Weitere matrixbildende Mono-, Oligo- und Polymere können in diese Haupt- und Seitenketten eingebunden sein, ohne selbst kovalent an die Trägerschicht gebunden zu sein.

Die Hauptkette kann zumindest teilweise polymerisierte Vinyl-, Allyl-, Acryl- oder Methacrylverbindungen oder deren Derivate und/oder deren Isomere oder auch Kombinationen davon umfassen.

Die Seitenketten umfassen insbesondere Mono- und/oder Oligosaccharide, wobei auch Reduktionsprodukte von Mono- oder Oligosacchariden als solche aufgefasst werden, insbesondere Zuckeralkohole (Alditole), sowie Peptide. Daneben können auch oxidierte Mono- und/oder Oligosaccharide auftreten, wobei auch die oxidierte Form im Sinne der Erfindung als Mono- oder Oligosaccharid aufgefasst wird.

Das erfindungsgemäße Medizinprodukt umfasst das Grundgerüst des Medizinprodukts als Substrat mit einer Beschichtung, wobei die Beschichtung bevorzugt eine auf dem Substrat befindliche Trägerschicht und eine auf der Trägerschicht befindliche Funktionsschicht umfasst. Die Trägerschicht umfasst im Wesentlichen die Haftvermittler, die kovalent an das Substrat gebunden sein können. Daneben sind auch nicht kovalent bindende Haftvermittler bekannt, beispielsweise solche, die über eine Komplexbindung an das Substrat binden. Bevorzugte Haftvermittler sind Siliciumverbindungen und polyolefinische Haftvermittler. Die Funktionsschicht umfasst gemäß einer bevorzugten Ausführungsform mindestens einen funktionalisierten Zuckeralkohol, über den die Funktionsschicht kovalent an die Trägerschicht gebunden ist, sowie ein funktionalisiertes Peptid.

Ein bevorzugter Zuckeralkohol der Funktionsschicht entspricht in seiner nicht funktionalisierten Form einem Zuckeralkohol mit der Summenformel C₆H₁₄O₆, beispielsweise Sorbitol, und/oder dessen Derivate, wie beispielsweise Sorbitan, und/oder dessen Isomere, wie beispielsweise Mannitol.

"In seiner nicht funktionalisierten Form" bedeutet, dass die angegebene Summenformel die Summenformel des nicht funktionalisierten Zuckeralkohols darstellt, jedoch gegebenenfalls auch dessen Derivate und/oder dessen Isomere umfasst sein sollen. Unter Funktionalisierung wird dabei die Einführung einer Funktion in die Verbindung verstanden, die eine Verknüpfung mit dem Substrat, der Trägerschicht und/oder bereits zuvor mit der Trägerschicht oder dem Substrat verbundenen Verbindungen erlaubt.

Die erfindungsgemäße Funktionsschicht kann auch funktionalisierte Varianten des Zuckeralkohols mit der Summenformel C₆H₁₄O₆ und/oder dessen Derivate und/oder dessen Isomere umfassen. Insbesondere kann die Funktionsschicht eine komplexe Matrix umfassen, die durch die Polymerisation der aufgebrachten, funktionalisierten Zuckeralkohole entstehen kann.

Die Zuckeralkohole der Funktionsschicht können zumindest teilweise untereinander polymerisiert sein.

Vorzugsweise umfasst die Beschichtung eine auf dem Substrat befindliche Trägerschicht, wobei die Funktionsschicht wiederum an die Trägerschicht gebunden ist. Bei den ausgebildeten Bindungen kann es sich insbesondere um kovalente, ggf. aber auch um andere Bindungen wie Komplexbindungen handeln. Die Trägerschicht umfasst im Wesentlichen die Haftvermittler, die an das Substrat gebunden sind. Bevorzugte Haftvermittler sind Siliciumverbindungen und polyolefinische Haftvermittler.

Ohne sich an eine bestimmte Theorie binden zu wollen, wird der Vorteil der erfindungsgemäßen Beschichtung hinsichtlich der Saccharide darin gesehen, dass die Funktionsschicht biomimetische bzw. biorepulsive Eigenschaften aufweist und von Thrombozyten nicht als körperfremd, sondern vielmehr als körpereigen erkannt wird. Entsprechend löst die erfindungsgemäße Funktionsschicht keine Reaktion der Thrombozyten aus, insbesondere keine Adhäsions- und auch keine Aggregationsreaktionen.

Der biomimetische Effekt der erfindungsgemäßen Beschichtung wird darauf zurückgeführt, dass die erfindungsgemäße Funktionsschicht die menschliche Glykokalyx nachahmt. Die Glykokalyx überzieht die Zellen der Blutgefäße mit einer Art Schleimschicht und besteht aus verschiedenen Polysacchariden, die kovalent an die Membranproteine (Glycoproteine) und Membranlipide (Glycolipide) gebunden sind.

Von Vorteil für den biomimetischen Effekt der erfindungsgemäßen Beschichtung - und insbesondere der Funktionsschicht - ist, dass die Polymerisation der Reaktanten der Funktionsschichtlösung im Wesentlichen erst nach dem Auftragen der Funktionsschichtlösung auf das Substrat bzw. die Trägerschicht stattfindet. Hierdurch entsteht durch Polymerisation der Reaktanten eine komplexe Schicht, die eine so hohe Ähnlichkeit zur Glykokalyx aufweist, dass die Anhaftung von Thrombozyten an mit der erfindungsgemäßen Beschichtung versehenen Flächen deutlich geringer ist als an unbeschichteten Oberflächen.

Der biorepulsive Effekt der erfindungsgemäßen Beschichtung wird auf das Prinzip der sterischen Repulsion zurückgeführt. Vermutlich wird der Raum, der den Oligo- und Polymeren auf der Oberfläche zur Verfügung steht, bei Annäherung eines Proteins reduziert, d. h. ein sich näherndes Protein zwingt die Oligo- und Polymere auf der Oberfläche, eine energetisch ungünstigere Konformation einzunehmen. Hierdurch ergibt sich insgesamt eine gegenüber Proteinen abstoßende Kraft. Möglicherweise führt auch die Verdrängung von Wassermolekülen aus der Beschichtung zu einer repulsiven osmotischen Kraft gegenüber Proteinen.

Für die Thrombozytenadhäsion bedeutet dieses Wirkungsprinzip, dass Thrombozyten nicht haften können, weil keine oder nur wenige zur Anbindung geeignete Proteine auf der Oberfläche vorhanden sind, wodurch die Thrombozytenadhäsion deutlich reduziert ist.

Auf der anderen Seite bewirkt die Gegenwart von Peptiden in der Funktionsschicht eine verstärkte Adhärenz von Endothelzellen am Medizinprodukt, bei dem es sich in der Regel um ein Implantat handelt, das für den dauerhaften Verbleib im Körper vorgesehen ist. Die verstärkte Zelladhärenz sorgt für eine rasche Endothelisierung und ein Einwachsen des Implantats in das umgebende Gewebe.

Vorteilhaft an der erfindungsgemäßen Beschichtung ist auch, dass die Beschichtung über den Zwischenschritt der Haftvermittlung nur solche Oberflächen und Strukturen des Medizinproduktes überzieht, die für die entsprechenden Haftvermittler aktivierbar sind und insbesondere auch aktiviert wurden. So ist es beim Aufbringen der Funktionsschichtlösung möglich, das komplette Medizinprodukt in die Funktionsschichtlösung einzubringen, ohne dass Bereiche zusätzlich geschützt werden müssten, die nicht beschichtet werden sollen.

Eine solche selektive Beschichtung bzw. ein in dieser Weise selektives Beschichtungsverfahren hat den zuvor beschriebenen Vorteil bei einer Vielzahl von Medizinprodukten, zumindest bei solchen, die verschiedene Materialien umfassen, eine Beschichtung jedoch nur auf bestimmten dieser Materialien erfolgen soll.

Die erfindungsgemäße Beschichtung lässt beim Beschichtungsprozess Möglichkeiten dafür, nur die Teile des Medizinprodukts zu aktivieren, die später auch die Funktionsschicht tragen sollen. Auch ist es denkbar, dass das Medizinprodukt bereits so konzipiert ist, dass für die zu beschichtenden Teile solche Stoffe gewählt werden, die aktivierbar für eine Haftvermittlung sind.

Medizinprodukte mit einer erfindungsgemäßen Beschichtung sind für den endovaskulären, insbesondere den neurovaskulären Einsatz und den kardiovaskulären Einsatz sowie den Einsatz im peripheren Bereich geeignet, jedoch kann die erfindungsgemäße Beschichtung für ein Medizinprodukt grundsätzlich immer dann sinnvoll sein, wenn das entsprechende Medizinprodukt mit Blut in Kontakt kommt.

Bei dem erfindungsgemäßen Medizinprodukt kann es sich beispielsweise um einen Stent (Gefäßendoprothese) handeln, wie sie zur Behandlung von Gefäßverengungen eingesetzt und an der Stelle der Gefäßverengung dauerhaft implantiert werden, um das Gefäß offen zu halten. Die Beschichtung bewirkt ein rasches Einwachsen des Implantats in das Gewebe, sodass es nach einer gewissen Zeit vom Körper nicht mehr als Fremdkörper wahrgenommen wird. Typischerweise weisen Stents eine Röhrenstruktur auf und sind entweder lasergeschnitten, so dass sich eine Oberfläche aus Streben ergibt, zwischen denen Öffnungen vorliegen, oder bestehen aus einem Drahtgeflecht. Stents können durch einen Katheter an den Zielort gebracht und dort expandiert werden.

Stentähnliche Implantate können auch zur Behandlung eines Vasospasmus in ein Blutgefäß implantiert werden. Unter einem Vasospasmus versteht man eine krampfartige Verengung eines Blutgefäßes. Hiermit ist die Gefahr verbunden, dass nachfolgende Gefäße nicht mehr in ausreichendem Maße mit Blut versorgt werden (Ischämie), was zur Nekrose des durch die Gefäße mit Blut versorgten Gewebes führen kann. Durch das Setzen eines Vasospasmusstents wird das Blutgefäß aufgeweitet.

Ein weiterer Anwendungsbereich der Beschichtung sind Flow Diverter, wie sie zur Behandlung von Aneurysmen eingesetzt werden. Hinsichtlich des grundsätzlichen Aufbaus und der Beschichtung gilt das zuvor gesagte, allerdings weist ein Flow Diverter typischerweise eine größere Oberflächenabdeckung bzw. Oberflächendichte als ein herkömmlicher Stent auf. Der Flow Diverter wird vor dem Aneurysmahals platziert, um dafür zu sorgen, dass der Blutstrom am Aneurysma vorbeigeleitet wird. Auf diese Weise wird dafür gesorgt, dass das Aneurysma letztlich verödet.

Eine weitere mögliche Funktion einer Stentstruktur bzw. eines Flow Diverters, die vor einem Aneurysma platziert werden, besteht darin, in das Aneurysma eingebrachte Okklusionsmittel wie z. B. Okklusionscoils daran zu hindern, aus dem Aneurysma zu entweichen. Ein solches Austreten von Okklusionsmitteln aus dem Aneurysma kann unerwünschte Folgen haben, wenn beispielsweise das Okklusionsmittel vom Blutstrom in weiter distal gelegene Bereiche getragen wird und dort für einen Verschluss des Blutgefäßes oder eine Verletzung der Blutgefäßwandung sorgt. Zu diesem Zweck kann die Stentstruktur dauerhaft im Blutgefäß implantiert werden.

Eine Untergruppe der Flow Diverter stellen sog. Bifurkationsflowdiverter dar, die vor Aneurysmen platziert werden, die sich an einer Gefäßverzweigung (Bifurkation) befinden. Ein solcher Bifurkationsflowdiverter bzw. Bifurkationsimplantat wird z. B. in der WO 2014/029835 A1 beschrieben. Ein solches Implantat weist einen distalen Abschnitt auf, der gegenüber einem weiter proximal angeordneten Abschnitt radial erweitert ist. Der distale Abschnitt ist so beschaffen, dass er den Hals des Aneurysmas mindestens teilweise verschließt. Auch für ein solches Bifurkationsimplantat ist die beschriebene Beschichtung zur Vermeidung von Thrombozytenadhäsion und -aggregation sinnvoll.

Die Begriffe "proximal" und "distal" sind so zu verstehen, dass beim Einbringen der Vorrichtung zum behandelnden Arzt weisende Teile als proximal, vom behandelnden Arzt weg weisende Teile als distal bezeichnet werden. Die Vorrichtung wird somit typischerweise durch einen Mikrokatheter in distaler Richtung vorgeschoben. Der Begriff "axial" bezieht sich auf die von proximal nach distal verlaufende Längsachse des Implantats, der Begriff "radial" auf hierzu senkrechte Ebenen.

Die beschriebenen Implantate können sich aus miteinander verbundenen Stegen bzw. Streben zusammensetzen. Eine solche Struktur kann durch Laserschneiden in grundsätzlich bekannter Weise hergestellt werden; man spricht in diesem Zusammenhang auch von geschnittenen Strukturen. Auf diese Weise wird am Implantat eine Vielzahl an Öffnungen bzw. eine Netzstruktur erzeugt, wobei die Öffnungen über den Umfang der Stentstruktur verteilt sind.

Auch andere Herstellungsverfahren sind denkbar, beispielsweise eine galvanische oder lithographische Herstellung, 3D-Druck oder Rapid Prototyping.

Alternativ können entsprechende Implantate sich auch aus einer Maschenstruktur aus Drähten aufbauen, die ein Geflecht ausbilden. Die Drähte verlaufen hierbei typischerweise helixförmig entlang der Längsachse, wobei gegenläufig verlaufende Drähte an den Kreuzungspunkten über- und untereinander her verlaufen, sodass sich wabenförmige Öffnungen zwischen den Drähten ausbilden. Die Gesamtzahl der Drähte beträgt bevorzugt 8 bis 64. Bei den Drähten, die die Maschenstruktur ausbilden, kann es sich um einzelne Drähte aus Metall handeln, möglich ist aber auch das Vorsehen von Litzen, d. h. mehreren Drähten geringen Durchmessers, die zusammen ein Filament bilden und vorzugsweise miteinander verdrillt sind.

Zumeist am proximalen Ende kann ein Implantat über eine Ablösestelle mit einer Einführhilfe, insbesondere einem Einführdraht verbunden sein. Die Ablösestelle kann z. B. elektrolytisch korrodierbar ausgebildet sein, sodass durch Anlegen einer elektrischen Spannung eine Ablösung des Implantats erfolgt. Ebenso sind auch andere aus dem Stand der Technik bekannte Ablösestellen verwendbar, insbesondere mechanisch, thermisch oder chemisch trennbare Ablösestellen. Bei einer mechanischen Ablösung besteht typischerweise ein Form-, Kraft- oder Reibschluss, der bei der Freisetzung des Implantats aufgehoben wird, sodass sich das Implantat von der Einführhilfe löst. Bei einer thermischen Ablösestelle kann die Verbindung dadurch aufgehoben werden, dass eine Erwärmung der Ablösestelle erfolgt, woraufhin diese so weich wird oder schmilzt, dass eine Abtrennung erfolgt. Schließlich ist auch eine chemische Ablösung möglich, bei der die Ablösung durch eine chemische Reaktion an der Ablösestelle herbeigeführt wird.

Das Implantat kann selbstexpandierend ausgebildet sein, d. h. es nimmt nach Freisetzung selbständig einen expandierten Zustand an. Hierzu wird das Implantat aus Materialien mit Formgedächtniseigenschaften gefertigt, beispielsweise Nickel-Titan-Legierungen, wie sie unter dem Namen Nitinol bekannt sind. Andere stentartige Implantate werden mit Hilfe eines Ballons aufgeweitet, auch für solche Implantate lässt sich die erfindungsgemäße Beschichtung verwenden.

Sinnvollerweise verfügt das Medizinprodukt über ein oder mehrere röntgendichte Markierungen, um dem behandelnden Arzt eine Visualisierung zu ermöglichen. Die röntgendichten Markierungen können z. B. aus Platin, Palladium, Platin-Iridium, Tantal, Gold, Wolfram oder anderen röntgendichten Metallen sein. Beispielsweise können röntgendichte Wendeln an verschiedenen Punkten des Medizinprodukts angebracht sein. Möglich ist auch, die Stentstruktur, insbesondere die Streben oder Drähte der Stentstruktur mit einer Beschichtung aus einem röntgendichten Material zu versehen, beispielsweise mit einer Goldbeschichtung. Diese kann z. B. eine Stärke von 1 bis 6 µm aufweisen. Die Beschichtung mit einem röntgendichten Material muss nicht das gesamte Medizinprodukt umfassen; von Bedeutung ist sie insbesondere in den Bereichen, die die Gefäßinnenwandung berühren, d. h. im Wesentlichen im zylindrischen Teil einer Stentstruktur. Auch beim Vorsehen einer röntgendichten Beschichtung kann es allerdings sinnvoll sein, zusätzlich einen oder mehrere röntgendichte Markierungen anzubringen, insbesondere am distalen Ende des Medizinprodukts.

Neben dem erfindungsgemäßen Medizinprodukt betrifft die Erfindung auch ein Verfahren zur Herstellung eines solchen Medizinprodukts, nämlich ein Verfahren zur Beschichtung eines Medizinprodukts mit einer Funktionsschicht, wobei die Funktionsschicht durch Reaktion von mit reaktiven Gruppen funktionalisierten Sacchariden und mit reaktiven Gruppen funktionalisierten Peptiden erzeugt wird, wobei die Reaktion in der Regel zumindest teilweise eine Polymerisation ist.

Sämtliche Ausführungen und beschriebenen Merkmalskombinationen zum Medizinprodukt gelten in entsprechender Weise auch für das erfindungsgemäße Verfahren und umgekehrt.

### Versuche

Es wurden *in vitro* Versuchsreihen mit der erfindungsgemäßen Beschichtung durchgeführt, um den Einfluss auf die Adhäsion von Endothelzellen einerseits und Thrombozyten andererseits zu untersuchen. Die untersuchten Nitinol-Probenplättchen wurden in 3 Gruppen aufgeteilt:
1) unbeschichtete Nitinol-Plättchen (A + D)
2) Nitinol-Plättchen mit einer Beschichtung von auf der Oberfläche polymerisierenden, mit polymerisierbaren Gruppen funktionalisierten Zuckeralkoholen (B + E)
3) Nitinol-Plättchen entsprechend der 2. Gruppe, die zusätzlich eine Beschichtung mit dem Peptid GRGDSPK erfahren haben (C + F).

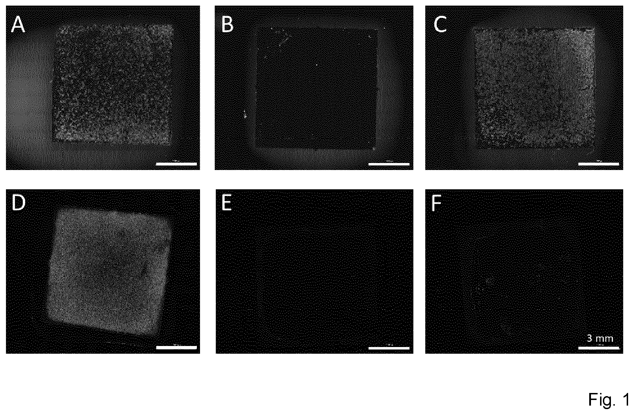

Das Ergebnis ist in Fig. 1 dargestellt. Die Plättchen A bis C wurden mit humanen Endothelzellen (HUVEC; human umbilical vein endothelial cells) besiedelt und mit einem spezifischen Fluoreszenzfarbstoff gefärbt. Die Plättchen D bis F wurden mit humanem Vollblut inkubiert. Adhärente Thrombozyten wurden mit einem spezifischen Fluoreszenzfarbstoff gefärbt.

Man erkennt, dass die unbeschichteten Plättchen A und D eine starke Anlagerung von Zellen zeigen, sowohl von Endothelzellen (A) als auch von Thrombozyten (D). Im Gegensatz dazu zeigen die Plättchen B und E, bei denen lediglich eine Saccharidschicht aufgebracht wurde, fast keine Anlagerung von Zellen, weder von Endothelzellen (B), noch von Thrombozyten (E). Die Abwesenheit von Thrombozyten ist auf der einen Seite positiv, um erneuter Thrombusbildung vorzubeugen, auf der anderen Seite wäre eine Adhärenz von Endothelzellen zur Förderung des Einwachsens eines Implantats wünschenswert.

Letzteres zeigen die Plättchen C und F. Die Besiedlung mit Endothelzellen (C) ist stark und liegt in einer vergleichbaren Größenordnung wie bei den unbeschichteten Plättchen. Die Anlagerung von Thrombozyten hingegen bleibt vernachlässigbar (F).

## Patentansprüche

1. Endovaskuläres Medizinprodukt, wobei mindestens Teile des Medizinprodukts eine Beschichtung tragen und die Beschichtung eine Funktionsschicht umfasst, welche Saccharide enthält, und wobei das Medizinprodukt ein für den dauerhaften Verbleib im Körper vorgesehenes Implantat ist,
**dadurch gekennzeichnet,**
**dass** die Funktionsschicht Peptidsequenzen mit Integrin-bindenden Motiven enthält.

2. Medizinprodukt nach Anspruch 1, dass die Funktionsschicht eine RGD-Peptidsequenz enthält.

3. Medizinprodukt nach Anspruch 1 oder 2, **dadurch gekennzeichnet, dass** die Funktionsschicht durch eine Reaktion von mit polymerisierbaren Gruppen funktionalisierten Sacchariden und mit reaktiven Gruppen funktionalisierten Peptiden mit Integrin-bindenden Motiven ausgebildet ist.

4. Medizinprodukt nach Anspruch 3, **dadurch gekennzeichnet, dass** die Funktionsschicht durch eine Copolymerisation von mit polymerisierbaren Gruppen funktionalisierten Sacchariden und mit reaktiven Gruppen funktionalisierten Peptiden mit Integrin-bindenden Motiven ausgebildet ist.

5. Medizinprodukt nach Anspruch 3, **dadurch gekennzeichnet, dass** die Funktionsschicht durch zeitlich aufeinander folgende Reaktionen von mit polymerisierbaren Gruppen funktionalisierten Sacchariden und mit reaktiven Gruppen funktionalisierten Peptiden mit Integrin-bindenden Motiven ausgebildet ist.

6. Medizinprodukt nach Anspruch 3, **dadurch gekennzeichnet, dass** die Funktionsschicht durch zeitlich aufeinander folgende Reaktionen von mit reaktiven Gruppen funktionalisierten Peptiden mit Integrin-bindenden Motiven und mit polymerisierbaren Gruppen funktionalisierten Sacchariden ausgebildet ist.

7. Medizinprodukt nach Anspruch 3, **dadurch gekennzeichnet, dass** die Funktionsschicht durch eine Polymerisation von polymerisierbaren Molekülen ausgebildet ist, welche eine Saccharideinheit und eine Peptideinheit tragen, wobei das Peptid über zumindest ein Integrin-bindendes Motiv verfügt.

8. Medizinprodukt nach einem der Ansprüche 1 bis 7, **dadurch gekennzeichnet, dass** die Saccharide Monosaccharide sind.

9. Medizinprodukt nach einem der Ansprüche 1 bis 8, **dadurch gekennzeichnet, dass** die Saccharide in ihrer nicht funktionalisierten Form mindestens teilweise Zuckeralkohole oder von Zuckeralkoholen durch Dehydratisierung ableitbare cyclische oder heterocyclische Verbindungen sind.

10. Medizinprodukt nach einem der Ansprüche 3 bis 9, **dadurch gekennzeichnet, dass** die polymerisierbaren Gruppen reaktive Mehrfachbindungen, insbesondere Doppelbindungen umfassen, wobei die Doppelbindungen insbesondere Bestandteil von (Meth)acryl-, Allyl oder VinylGruppen sind.

11. Medizinprodukt nach einem der Ansprüche 1 bis 10, **dadurch gekennzeichnet, dass** die Beschichtung eine Trägerschicht mit einem Haftvermittler umfasst und die Funktionsschicht an die Trägerschicht gebunden ist.

12. Medizinprodukt nach Anspruch 11, **dadurch gekennzeichnet, dass** die Bindung des Haftvermittlers an das Medizinprodukt und/oder die Bindung der Funktionsschicht an die Trägerschicht eine kovalente Bindung ist.

13. Medizinprodukt nach Anspruch 11 oder 12, **dadurch gekennzeichnet, dass** der Haftvermittler eine Siliciumverbindung, insbesondere eine Silanverbindung, umfasst.

14. Medizinprodukt nach einem der Ansprüche 3 bis 13, **dadurch gekennzeichnet, dass** zwischen der polymerisierbaren Gruppe und dem Saccharid und/oder Peptid ein Spacer vorhanden ist.

15. Verfahren zur Herstellung eines Medizinprodukts nach einem der Ansprüche 1 bis 14 durch Beschichtung eines Medizinprodukts mit einer Funktionsschicht, wobei die Funktionsschicht durch Reaktion von mit reaktiven Gruppen funktionalisierten Sacchariden und mit reaktiven Gruppen funktionalisierten Peptiden mit Integrin-bindenden Motiven erzeugt wird.

## Claims

1. Endovascular medical device, wherein at least parts of the medical device are coated and the coating comprises a functional layer containing saccharides and wherein the medical device is an implant intended to remain in the body permanently,
**characterised in that**
the functional layer contains peptide sequences with integrin-binding motifs.

2. Medical device according to claim 1, in that the functional layer contains an RGD peptide sequence.

3. Medical device according to claim 1 or 2, **characterised in that** the functional layer is formed by a reaction of saccharides functionalised with polymerisable groups and peptides functionalised with reactive groups with integrin-binding motifs.

4. Medical device according to claim 3, **characterised in that** the functional layer is formed by a copolymerisation of saccharides functionalised with polymerisable groups and peptides functionalised with reactive groups with integrin-binding motifs.

5. Medical device according to claim 3, **characterised in that** the functional layer is formed by successive reactions of saccharides functionalised with polymerisable groups and peptides functionalised with reactive groups with integrin-binding motifs.

6. Medical device according to claim 3, **characterised in that** the functional layer is formed by successive reactions of peptides functionalised with reactive groups with integrin-binding motifs and saccharides functionalised with polymerisable groups.

7. Medical device according to claim 3, **characterised in that** the functional layer is formed by a polymerisation of polymerisable molecules carrying a saccharide unit and a peptide unit, wherein the peptide has at least one integrin-binding motif.

8. Medical device according to any one of claims 1 to 7, **characterised in that** the saccharides are monosaccharides.

9. Medical device according to any one of claims 1 to 8, **characterised in that** the saccharides in their non-functionalised form are at least partly sugar alcohols or cyclic or heterocyclic compounds derivable from sugar alcohols by dehydration.

10. Medical device according to any one of claims 3 to 9, **characterised in that** the polymerisable groups comprise reactive multiple bonds, in particular double bonds, wherein the double bonds are in particular components of (meth)acrylic, allyl or vinyl groups.

11. Medical device according to any one of claims 1 to 10, **characterised in that** the coating comprises a carrier layer with an adhesion promoter and the functional layer is bonded to the carrier layer.

12. Medical device according to claim 11, **characterised in that** the bonding of the adhesion promoter to the medical device and/or the bonding of the functional layer to the carrier layer is a covalent bond.

13. Medical device according to claim 11 or 12, **characterised in that** the adhesion promoter comprises a silicon compound, in particular a silane compound.

14. Medical device according to any one of claims 3 to 13, **characterised in that** a spacer is provided between the polymerisable group and the saccharide and/or peptide.

15. Method for producing a medical device according to any one of claims 1 to 14 by coating a medical device with a functional layer, wherein the functional layer is produced by the reaction of saccharides functionalised with reactive groups and peptides functionalised with reactive groups with integrin-binding motifs.

## Revendications

1. Produit médical endovasculaire, au moins des parties du produit médical portant un revêtement et le revêtement comprenant une couche fonctionnelle qui contient des saccharides, et le produit médical étant un implant destiné à rester en permanence dans le corps,
**caractérisé en ce que**
la couche fonctionnelle contient des séquences peptidiques avec des motifs de liaison à l'intégrine.

2. Produit médical selon la revendication 1, **caractérisé en ce que** la couche fonctionnelle contient une séquence peptidique RGD.

3. Produit médical selon la revendication 1 ou 2, **caractérisé en ce que** la couche fonctionnelle est formée par une réaction de saccharides fonctionnalisés par des groupes polymérisables et de peptides fonctionnalisés par des groupes réactifs avec des motifs de liaison à l'intégrine.

4. Produit médical selon la revendication 3, **caractérisé en ce que** la couche fonctionnelle est formée par une copolymérisation de saccharides fonctionnalisés par des groupes polymérisables et de peptides fonctionnalisés par des groupes réactifs avec des motifs de liaison à l'intégrine.

5. Produit médical selon la revendication 3, **caractérisé en ce que** la couche fonctionnelle est formée par des réactions successives dans le temps de saccharides fonctionnalisés par des groupes polymérisables et de peptides fonctionnalisés par des groupes réactifs avec des motifs de liaison à l'intégrine.

6. Produit médical selon la revendication 3, **caractérisé en ce que** la couche fonctionnelle est formée par des réactions successives dans le temps de peptides fonctionnalisés par des groupes réactifs avec des motifs de liaison à l'intégrine et de saccharides fonctionnalisés par des groupes polymérisables.

7. Produit médical selon la revendication 3, **caractérisé en ce que** la couche fonctionnelle est formée par une polymérisation de molécules polymérisables portant une unité saccharide et une unité peptide, le peptide disposant d'au moins un motif de liaison à l'intégrine.

8. Produit médical selon l'une quelconque des revendications 1 à 7, **caractérisé en ce que** les saccharides sont des monosaccharides.

9. Produit médical selon l'une quelconque des revendications 1 à 8, **caractérisé en ce que** les saccharides, sous leur forme non fonctionnalisée, sont au moins partiellement des alcools de sucre ou des composés cycliques ou hétérocycliques dérivables d'alcools de sucre par déshydratation.

10. Produit médical selon l'une quelconque des revendications 3 à 9, **caractérisé en ce que** les groupes polymérisables comprennent des liaisons multiples réactives, notamment des doubles liaisons, les doubles liaisons faisant notamment partie de groupes (méth)acryliques, allyliques ou vinyliques.

11. Produit médical selon l'une quelconque des revendications 1 à 10, **caractérisé en ce que** le revêtement comprend une couche de support avec un promoteur d'adhésion et la couche fonctionnelle est liée à la couche de support.

12. Produit médical selon la revendication 11, **caractérisé en ce que** la liaison du promoteur d'adhésion au produit médical et/ou la liaison de la couche fonctionnelle à la couche de support est une liaison covalente.

13. Produit médical selon la revendication 11 ou 12, **caractérisé en ce que** le promoteur d'adhésion comprend un composé de silicium, notamment un composé de silane.

14. Produit médical selon l'une quelconque des revendications 3 à 13, **caractérisé en ce qu'**un espaceur est présent entre le groupe polymérisable et le saccharide et/ou le peptide.

15. Procédé de fabrication d'un produit médical selon l'une quelconque des revendications 1 à 14 par revêtement d'un produit médical avec une couche fonctionnelle, la couche fonctionnelle étant produite par réaction de saccharides fonctionnalisés par des groupes réactifs et de peptides fonctionnalisés par des groupes réactifs avec des motifs de liaison à l'intégrine.
